(19) Europäisches Patentamt
European Patent Office
Office européen des brevets

(11) Publication number: **0 146 823**
**B1**

(12) **EUROPEAN PATENT SPECIFICATION**

(45) Date of publication of patent specification: **25.03.87**

(51) Int. Cl.$^4$: **C 07 C 51/353**, C 07 C 53/08, C 07 C 53/122, C 07 C 53/124 // B01J23/46

(21) Application number: **84114569.1**

(22) Date of filing: **30.11.84**

(54) **Production of carboxylic acids from organic formate esters.**

(30) Priority: **02.12.83 US 557270**

(43) Date of publication of application:
**03.07.85 Bulletin 85/27**

(45) Publication of the grant of the patent:
**25.03.87 Bulletin 87/13**

(84) Designated Contracting States:
**DE FR GB IT NL**

(56) References cited:
**DE-A-2 236 439**
**DE-A-3 046 899**
**GB-A-1 286 224**

(73) Proprietor: **UNION CARBIDE CORPORATION**
**39 Old Ridgebury Road**
**Danbury Connecticut 06817 (US)**

(72) Inventor: **Wegman, Richard William**
**912 Glendale Avenue**
**South Charleston 25303 (US)**
Inventor: **Busby, David Charles**
**5323 Edgebrook Road**
**Cross Lanes 25313 (US)**
Inventor: **Schreck, David James**
**5226 Sun Valley Drive**
**Cross Lanes 25313 (US)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

**EP 0 146 823 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

The production of organic compounds using carbon monoxide or synthesis gas, which is a mixture of carbon monoxide and hydrogen, as reacant has been known for a significant period of time. It is well known that one can produce methanol directly from synthesis gas and that methanol can be further reacted by hydroformylation, homologation and carbonylation reactions to produce acetaldehyde, ethanol and acetic acid or its methyl ester, respectively. It is also known that esters, ethers, and other organic compounds can be reacted with carbon monoxide or synthesis gas to produce oxygenated organic compounds. The difficulties, however, have resided in the ability to carry out any one of these chosen reactions to produce the desired compound at acceptable efficiency conversion rate and selectivity.

In almost all instances the reaction is generally catalyzed using a Group VIII transition metal compound as catalyst and with a halogen as the promoter. It is known that many other metal compounds and promoters can be used. In addition, the prior art has disclosed the use of secondary activators or ligands in conjunction with the metal catalysts and promoters. These secondary activators can be other metallic salts or compounds, amines, phosphorus compounds, as well as a multitude of other compounds that have been disclosed in the published literature. Thus, a typical catalyst system contains the metal atom catalyst, promoter and, optionally, ligands, solvents and secondary activators. Though a significant amount of literature does exist describing the production of acetic acid by the isomerization of methyl formate, to our knowledge it does not disclose or suggest our invention. Several of the pertinent patents in this area are discussed below.

FR—A—2,317,269 discloses the production of aliphatic carboxylic acids by the reaction of an alcohol with carbon monoxide in the presence of a catalyst containing at least three essential components, iridium atom, copper atom and halogen. This is not our process.

In EP—A1—0,018,927 there is described a process for the production of monocarboxylic acids by the carbonylation of an alcohol using a nickel catalyst, a halide and a solvent; in this reference synthesis gas is used. In the process of this invention of this instant application an organic acid is produced from a formate ester using a rhodium atom catalyst and lithium iodide.

EP—A1—0,045,637 discloses the direct conversion of formic acid esters to their corresponding carboxylic acids without the presence of carbon monoxide using as catalyst a soluble iridium salt and an iodine promoter. This is not our catalytic process.

Another known procedure for producing acetic acid is the catalytic isomerization of methyl formate as shown by the reaction:

$$CH_3OOCH \rightarrow CH_3COOH$$

This procedure is shown in US—A—1,697,109. The process described is a vapor phase isomerization reaction carried out at 200°C to 450°C at a pressure up to 200 atmospheres using a metal oxide or acetate catalyst. It does not disclose the use of rhodium and lithium iodide.

U.S.—A—2,508,513 claims discloses the carbonylation of e.g. nickel, promoted with methyl iodide for the isomerization of methyl formate to acetic acid, carried out at 300°C to 400°C and a pressure up to 400 atmospheres. Carbon monoxide may be present. It does not disclose the use of rhodium plus lithium iodide.

U.S.—A—3,060,233 discloses the carbonylation of methanol to acetic acid using a metal of the iron group of the Periodic Table and a halide. It does not disclose use of rhodium or the use of a formate ester.

U.S.—A—2,769,329 discloses the production of carboxylic acids from alcohols, or the ester, ether and halide derivatives thereof, and carbon monoxide using a thodium catalyst and a halogen component. It does not mention lithium iodide.

U.S.—A—2,798,267 relates to the conversion of methyl formate to acetic acid in the presence of a catalyst system consisting essentially of activated carbon and a halogen promoter. The reference uses catalyst and starting materials different than those employed in the invention of this application.

U.S.—A—4,194,056 discloses the production of carboxylic acid from methyl formate using a soluble rhodium catalyst, halogen promoter and carbon monoxide. This is not the process of the instant invention, nor does this reference suggest or disclose the use of lithium iodide and the unexpected results achieved by its use.

U.S.—A—4,212,989 (DE—A1—3,046,899) describes a process for producing carboxylic acids or their esters by reacting an alcohol or an ether with carbon monoxide using a Group VIII metal catalyst and an iodine promoter. The reference contains no suggestion or disclosure of the production of organic carboxylic acids from a formate ester.

GB—A—1,286,224 relates to the reaction of methyl formate with carbon monoxide in contact with a rhodium catalyst and a halogen promoter to produce acetic acid. It contains no recognition of the distinct advantages achieved with the use of lithium iodide, in fact it does not mention this specific compound.

GB—A—1,293,193 relates to the direct conversion of formic acid esters to the corresponding carboxylic acids, in the presence of carbon monoxide, a catalyst that is a Group IIb or VIII metal and an organic polar solvent. It does not disclose use of rhodium atom plus lithium iodide.

JP—A1—50—16773 discloses the production of an organic acid from the corresponding formic acid

ester in the presence of carbon monoxide using a catalyst system containing cobalt, iron or mercury and a halogen plus an alkali metal salt of a lower aliphatic carboxylic acid, triamine or cyclic amine.

JP—A1—51—65703 discloses the reaction of methyl formate in the presence of carbon monoxide using a system containing a rhenium catalyst and halogen compound to produce acetic acid.

JP—A1—56—22745 discloses the isomerization of a formic acid ester to the corresponding acid in the presence of carbon monoxide, palladium atom, halogen and base.

JP—A1—56—73040 relates to a process for producing acetic acid by isomerizing methyl formate in the presence of carbon monoxide using a nickel catalyst, an iodine compound and an organic nitrogen compound.

JP—A1—56—83439 discloses a method for producing acetic acid by heating methyl formate and carbon monoxide in contact with a catalyst containing palladium, ruthenium and/or irridium metal atom and a halide promoter.

None of the 5 Japanese Patent Applications disclose a process for producing acetic acid from a formate ester using a catalyst mixture consisting essentially of rhodium metal atom and lithium iodide.

It can be seen that the prior art contains many disclosures dealing with the catalytic production of acetic acid, including its production by the isomerization of methyl formate. The art also discloses the production of other organic carboxylic acids by the isomerization of other formate esters. One of the disadvantages in many of these references is the presence of water with the eventual need to remove it from the desired organic acid product. This removal is both complicated and costly. Other disadvantages often include the simultaneous occurrence of other reactions leading to the formation of by-products, such as, dimethyl acetal, methyl acetate, ethanol, etc. These reactions compete with the organic acid production resulting in low conversion rate and selectivity to organic acid.

Many processes employed for the production of organic acids use a catalyst system containing a source of metal atom and a source of halide atom. The alkali metal halides are often mentioned as suitable halide sources, but no distinction is made between any specific one of the alkali metal halides or between any other halogen compound.

## Summary of the Invention

A catalyst system and process for the production of organic acids at high efficiency, selectivity and conversion rate by the reaction of mixtures of an organic esters of formic acid, such as methyl formate, with the presence of carbon monoxide or synthesis gas has been found. The catalyst system charged to the reactor in the process contains rhodium atoms, lithium iodide or bromide and optionally an organic ligand. The use of lithium iodide in this system within the ranges defined results in unexpectedly high efficiency, high conversion rate or activity and high selectivity not heretofore achieved.

## Description of the Invention

In the catalytic reactions of synthesis gas or carbon monoxide in process to produce oxygenated organic compounds there are several criteria required of the catalyst. The catalyst must be as stable as possible, it should have a high activity or conversion rate, and it should have as high a selectivity for the desired product as possible.

Stability of the catalyst relates to how long the catalyst remains functional before either breaking down or losing its catalytic effect.

Activity or conversion rate relates to the amounts of reactants the catalyst converts to product per unit of time, generally expressed in g. mole per liter per hour (g mole/l·h).

Selectivity relates to the quantity of desired product produced, generally expressed in mole percent, based on the total amount of both desired products and undesired products produced.

The goal to be achieved is high values for all three criteria and continued efforts are being made to find new catalyst compositions to reach this goal without having a significant detrimental effect on the overall process. Toward this goal the prior art has developed catalyst systems containing a wide variety of metal atoms, promoters and activators, in many cases with diverse other components added. Though these catalyst systems are effective, improvement is always desirable.

The present invention is based on the unexpected and unpredictable discovery that the rhodium-lithium iodide/bromide system is an unexpectedly superior catalytic system for the production of organic acids from esters of formic acid (hereinafter "formate") at unexpected high efficiency, selectivity and conversion rate. It was also found that a ligand, $ER_3''$, can also be present as an optional component of the system. This unexpected improvement in efficiency, selectivity and conversion rate is achieved when the system's components are maintained within a defined range and when lithium iodide/bromide is present as the source of the halogen component in the system. Optionally a solvent and/or diluent can also be present. The improved catalyst system of this invention can be portrayed as containing the components Rh—LiI—$Er_3''$, wherein Rh is the rhodium containing compound and $ER_3''$ is optionally present.

In the process of our invention formates are reacted, with carbon monoxide or synthesis gas, using a particular catalyst system containing rhodium atoms and lithium iodide or bromide. This system produces commercially desirable organic acids at unexpectedly high efficiency, conversion rate and selectivity, with a minimum of by-products and without the presence of water. The overall reaction that occurs is theoretically:

$HCOOR \rightarrow RCOOH$

In the above formula R is a monovalent hydrocarbyl group. It can be an alkyl group having from 1 to 30 carbon atoms, preferably from 1 to 15 carbon atoms, and most preferably from 1 to 5 carbon atoms; an alkenyl group having from 2 to 30 carbon atoms, preferably from 2 to 15 carbon atoms and most preferably from 2 to 5 carbon atoms; or an aryl, aralkyl or alkaryl group hving 6 or 10 ring carbon atoms, e.g., phenyl and naphthyl, with from 1 to 10 carbon atoms, preferably 1 to 4 carbon atoms, in the alk-moiety thereof. The R group can be linear or branched and it can be unsubstituted or substituted with groups which will not have an adverse effect on the reaction; further; the alkenyl groups can contain more than one unsaturated bond.

Illustrative of suitable formic acid esters one can mention methyl formate, ethyl formate, the propyl formates, the butyl formates, the decyl formates, 2-ethylhexyl formate, stearyl formate, phenyl formate, benzyl formate, vinyl formate, allyl formate, naphthyl formate, tolyl formate.

The rhodium component of the catalyst system can be supplied from any number of sources, many of these are known to those of ordinary skill in the art. Thus, it is not necessary for an understanding thereof to specifically enumerate every suitable type and every specific compound since any of the known rhodium compounds can be used.

The essential rhodium component of the catalyst system of the present invention may be provided by introducing into the reaction zone a compound of rhodium or may be provided by introducing into the reaction zone rhodium. Among the materials which may be charged to the reaction zone to provide the rhodium component of the catalyst system of the present invention are rhodium metal, rhodium salts and oxides, organo rhodium compounds, and coordination compounds of rhodium. Specific examples of materials capable of providing the rhodium constituent of the catalyst system of the present invention may be taken from the following list of suitable materials.

$RhCl_2$
$RhBr_3$
$RhI_2$
$RhCl_3\ 3H_2O$
$RhBr_33H_2O$
$Rh_2(CO)_4Cl_2$
$Rh_2(CO)_4Br_2$
$Rh_2(CO)_4I_2$
$Rh_2(CO)_8$
$Rh[(C_6H_5)_3P]_2(CO)I$
$Rh[(C_6H_5)_3P]_2(CO)Cl$
Rh metal
$Rh(NO_3)_3$
$RhCl[(C_6H_5)_3P]_2(CH_3I)_2$
$Rh(SnCl_3)[(C_6H_5)_3P]_2$
$RhCl(CO)[(C_6H_5)_3As]_2$
$RhI(CO)[(C_6H_5)_3Sb]_2$
$[(n\text{-}C_4H_9)_4N][Rh(CO)_2X_2]$where X=Cl-, Br-, I-
$[(n\text{-}C_4H_9)_4AS]_2[Rh(CO)_2Y_4]$where X=Br-, I-
$[(n\text{-}C_4H_9)_4P][Rh(CO)I_4]$
$Rh[(C_6H_5)_3P]_2(CO)Br$
$Rh[(n\text{-}C_4H_9)_3P]_2(CO)Br$
$Rh[(n\text{-}C_4H_9)_3P]_2(CO)I$
$RhBr[(C_6H_5)_3P]_3$
$RhI[(C_6H_5)_3P]_3$
$RhCl[(C_6H_5)_3P]_2$
$RhCl[(C_6H_5)_3P]_3H_2$
$[(C_6H_5)_3P]_3Rh(CO)H$
$Rh_2O_3$
$[Rh(C_3H_4)_2Cl]_2$
$K_4Rh_2Cl_2(SnCl_2)_4$
$K_4Rh_2Br_2(SnBr_3)_4$
$K_4Rh_2I_2(SnI_2)_4$

The rhodium concentration can vary over a wide range. Enough metal atom must be present to achieve reasonable reaction rates; however, an excess may on occasion result in undesired by-products formation. The mole ratio of rhodium to formate can vary from 1:25 to 1:40,000, the preferred range is from 1:40 to 1:5,000, with the most preferred range being from 1:100 to 1:2,000. The amount used is not a critical feature in this invention and higher rhodium concentrations are acceptable but are influenced by economic considerations.

The second component of the catalyst system is lithium iodide or bromide. It can be charged directly, or it can be formed in situ by any combination of lithium compound and iodine component that will result in the formation of lithium iodide/bromide during the reaction. Lithium bromide can be used but the iodide is preferred. The presence of lithium iodide or lithium bromide is a critical feature of this invention. Direct charge of lithium iodide is the preferred form. However, any convenient combination of compounds for in situ formation of lithium iodide can be used. This includes the use of lithium carboxylates, carbonates and the like with a halogen compound such as iodine or bromine or an alkyl halide. A suitable combination for in situ formation is lithium carboxylate and an alkyl halide.

Sufficient lithium iodide/bromide must be present to exert a promoting effect on the reaction and to result in high efficiency, conversion rate and selectivity to the corresponding organic acid. The mole ratio of Rh:LiI (Br) can vary over a wide range. A Rh:LiI mole ratio of from 1:1 to 1:1000 can be employed, the preferred range is from 1:2 to 1:450 and most preferably it is from 1:8 to 1:150.

As indicated, an organic ligand of the general formula $ER_3''$ can optionally be present in the reaction system. The use of such ligands is known, as are their identities, to those skilled in this art. In this formula E represents a Group VA element, e.g., N, P, As, Sb and Bi, and R'' represents an organic moiety. The ligand can serve as a catalyst stabilizer and/or to further enhance efficiency, conversion rate and selectivity, especially when the reaction is carried out at higher temperatures, for example at 200°C or above. The ligand also serves to inhibit equipment corrosion in some instances. However, the use of a ligand is not mandatory and the reaction can be carried out without it.

A large number of organic ligands is known and any of these can be used provided they do not have an adverse effect on the reaction. Among those of particular utility are the tertiary amines and the tri- and pentavalent phosphorus compounds. Though those skilled in the art know these compounds, illustrative of suitable compounds one can mention triethylphosphine, tributylphosphine, tri-2-ethylhexylphosphine, triphenylphosphine, tri(4-methoxyphenyl)phosphine, tri-p-tolylphosphine, tri(3-chlorophenyl)phosphine, diphenyl hexylphosphine, dimethyl (3-methoxyphenyl)phosphine, dibutyl stearylphosphine, tribenzylphosphine, dipropyl phenylphosphine, ethyl dipropylphosphine, tricyclohexylphosphine, cyclohexyl dibutylphosphine, propyl diphenylphosphine, dipropyl phenylphosphine, phenyl diethylphosphine, tridecylphosphine, trioctadecylphosphine, tribenzylphosphine, methyl diethylphosphine, ethyl diphenylphosphine, tolyl diethylphosphine, cyclohexyl diethylphosphine, diethyl cyclohexylphosphine, bis-(diphenylphosphino)-ethane, bis-(diethylphosphino)-propane, bis-(diphenylphosphino)-butane, bis-(diethylphosphino)-octane, trimethylamine, triethylamine, tri-n-butylamine, tri-t-butylamine, tri-2-ethylhexylamine, methyl dibutylamine, tridodecylamine, tristearylamine, ethyl dibutylamine, tricyclohexylamine, triphenylamine, tri(4-methoxyphenyl)amine, tri(p-chlorophenyl)-amine, dibutyl phenylamine, dipentyl cyclopentylamine, ethyl diphenylamine, trinaphthylamine, tri-p-tolylamine, tri-benzylamine, tri(3-methylcyclohexyl)-amine, and the arsines, stibines and bismuthines corresponding to the above-identified phosphines and amines. They can be used singly or, if one desires, mixtures containing two or more ligands can be used. One can also employ a phosphine oxide or phosphite corresponding to the above phosphines as the ligand; these are also well known.

The concentration of ligand charged can vary from a molar ratio of ligand to rhodium of from 50:1 to 1:50, preferably from 10:1 to 1:10, most preferably 3:1 to 1:1.

In addition to the ligand one can optionally have a solvent present. Many essentially inert solvents are known as useful diluents and illustrative thereof one can mention 1,4-dioxane, the polyethylene glycol diethers or esters, diphenyl ether, sulfolane, toluene, propanol, carboxylic acids as well as any other diluent or solvent which does not interfere with the reaction to any significant extent. The reaction is preferably carried out in the absence of any solvent or diluent other than those required to introduce reactants or catalyst components.

The reaction is carried out at a temperature of from 50°C to 350°C, preferably from 120°C to 220°C and most preferably from 140°C to 200°C. When the reaction is carried out at temperatures above 200°C in the presence of an $ER_3''$ ligand, the preferred ligands are the phosphines, especially triphenyl phosphine.

The pressure of the reaction can be from 10.35 to 690 bar (150 psig to 10,000 psig), preferably from 13.8 to 69 bar (200 psig to 1,000 psig), most preferably from 13.8 to 34.5 bar (200 psig to 500 psig).

The reaction time varies depending upon the reaction parameters, reactor size and charge, and the individual components employed at the specific process conditions. The reaction can be a batch or continuous reaction.

The experiments and examples detailed below were carried out in a Hasteloy® steel autoclave reactor having a volume of 300 ml, which was equipped with temperature and pressure sensing means, heating and cooling means, agitator and inlet and outlet means for introducing and removing components from the reactor. The autoclaves used in synthesis gas reactions are well known in the art and can be used in this process.

Prior to charging the reactants the autoclave was washed with methanol at 100°C under a nitrogen gas pressure of 34.5 to 69 bar (500 to 1,000 psig) by agitating for 30 minutes. The autoclave was drained, rinsed with dry acetone, and dried with nitrogen. The liquid components were charged to the cleaned autoclave first and then the solid components were added and stirred. The autoclave was closed and purged with synthesis gas and then pressurized to the desired pressure with carbon monoxide or synthesis gas. The autoclave contents were heated to the selected temperature, with agitation (usually 750 rpm), in about 45

minutes. After the desired temperature was reached, the reaction was allowed to consume gas for the time period indicated. During this time the pressure was maintained by addition of gas as needed.

At the end of the reactor run, the contents were cooled, generally to about 10°C. A vapor phase sample was taken for gas chromatography analysis; the gas phase was vented through 2 dry-ice acetone traps and then through a 10 liter saturated solution of calcium hypochlorite to remove metal carbonyls, is formed. The reactor was pressurized 3 times with nitrogen, 6.2 bar (90 psig), and vented through the same system.

The residual reactor contents were dumped into a chilled pressure bottle and sealed. Subsequent analysis was performed using a Hewlett-Packard Model 5880 gas chromatograph equipped with a 3.2 mm diameter by 3 m long column packed with Chromosorb 101.

The following examples serve to further illustrate this invention. In the examples the term "AcAc" means "acetylacetonate".

Example 1

The autoclave was charged with 2.06 g of $Rh(CO)_2AcAc$ (8 mmoles), 8.57 g of lithium iodide (64 mmoles) and 146 g of methyl formate (2.45 moles). Following the procedure described above carbon monoxide was added to raise the reactor pressure to 27.6 bar. The temperature was raised to 180°C, reactor pressure was 49.7 bar and the reaction was continued for 3 hours at this pressure. The reactor was then cooled and treated as described above. Analysis indicated the following liquid products present in the reaction product.

| | |
|---|---|
| Acetic acid | 2.30 moles |
| Formic acid | 0.01 mole |
| Methyl acetate | 0.01 mole |
| Methyl formate | Trace |

The conversion rate and selectivity to acetic acid was 5.1 gmole/l·h; methyl formate conversion was 99%.

The data shows the high efficiency, conversion rate and selectivity achieved by the process of this invention under relatively mild pressure and temperature conditions.

Example 2

A series of runs was carried out in which the concentratio of lithium iodide charged was varied. The reaction conditions and recovery procedures were those described in Example 1. The reactants and results are summarized in Table I:

TABLE I

| Run | A | B | C | D |
|---|---|---|---|---|
| **Reactor Charge** | | | | |
| $Rh(CO)_2$ AcAc, mmoles | | 8 | | |
| LiI, mmoles | 16 | 32 | 64 | 128 |
| Methyl formate, moles | | 2.45 | | |
| **Products Recovered (Liquid)** | | | | |
| Acetic acid, moles | 0.15 | 1.34 | 2.28 | 2.4 |
| Formic acid, moles | 0.04 | 0.01 | 0.01 | 0.04 |
| Methyl acetate, moles | 0.04 | 0.01 | 0.01 | 0.04 |
| Methyl formate, moles | 1.97 | 0.44 | 0.01 | 0 |
| **Analytical Results** | | | | |
| Methyl formate conversion, % | 20 | 84 | 99.9 | 99.9 |
| Conversion rate, gmoles/l·h | 0.3 | 2.6 | 5.1 | 16.6 |
| Selectivity, mole % | 65 | 85 | 99.1 | 99.1 |

The results show that increasing the lithium iodide concentration increased the methyl formate conversion, conversion rate and selectivity.

Example 3

A series of runs was carried out in which the concentration of the rhodium atom charged was varied. The reaction conditions and recovery procedures were those described in Example I. The reactants and results are summarized in Table II.

TABLE II

| Run | A | B* | C | D | E |
|---|---|---|---|---|---|
| **Reactor Charge** | | | | | |
| $Rh(CO)_2$ AcAc, mmoles | 8 | 4 | 2 | 1 | 0.5 |
| LiI, mmoles | ← | | 64 | | → |
| Methyl formate, moles | ← | | 2.45 | | → |
| **Products Recovered (Liquid)** | | | | | |
| Acetic acid, moles | 2.32 | 1.88 | 1.98 | 0.66 | 0.33 |
| Formic acid, moles | 0.01 | 0.02 | 0.23 | 0.16 | 0.06 |
| Methyl acetate, moles | 0.01 | 0.02 | 0.23 | 0.16 | 0.06 |
| Methyl formate moles | 0 | 0 | 0.4 | 1.48 | 1.92 |
| **Analytical Results** | | | | | |
| Methyl formate conversion % | 99 | 99 | 94 | 39 | 20 |
| Conversion rate gmoles/l·h | 5.1 | 4.2 | 4.4 | 1.5 | 0.7 |
| Selectivity, moles % | 99.1 | 97.9 | 82 | 68.7 | 71.4 |

* Some product loss during recovery.

The results show the decrease in conversion, rate and selectivity with decreasing rhodium concentration.

Example 4

A series of runs was carried out in which the initial carbon monoxide pressure to the autoclave was varied. The other reaction conditions and the recovery procedures were those described in Example 1. The reactants and results are summarized in Table III.

TABLE III

| Run | A | B | C | D | E | F | G |
|---|---|---|---|---|---|---|---|
| Reactor Charge | | | | | | | |
| Reactor temperature, °C | 140 | 140 | 140 | 180 | 180 | 180 | 180 |
| $Rh(CO)_2$ AcAc, mmoles | ← | | | 8 | | | → |
| LiI, mmoles | 64 | 64 | 64 | 32 | 32 | 32 | 32 |
| Methyl formate, moles | ← | | | 2.45 | | | → |
| CO, initial pressure, (psig) | (400) | (600) | (800) | (100) | (200) | (300) | (400) |
| bar | 27.6 | 41.4 | 55.2 | 6.9 | 13.8 | 20.7 | 27.6 |
| Products Recovered (Liquid) | | | | | | | |
| Acetic acid, moles | 0.61 | 0.54 | 0.51 | 0.1 | 1.49 | 1.58 | 1.34 |
| Formic acid, moles | 0.03 | 0.03 | 0.03 | 0.02 | 0.15 | 0.12 | 0.1 |
| Methyl acetate, moles | 0.03 | 0 | 0.03 | 0.02 | 0.15 | 0.12 | 0.1 |
| Methyl formate, moles | 1.65 | 1.72 | 1.72 | 2.12 | 0.46 | 0.51 | 0.44 |
| Analytical Results | | | | | | | |
| Methyl formate conversion, % | 32 | 30 | 30 | 71 | 84 | 87 | 86 |
| Conversion rate, gmoles/l·h | 1.3 | 1.2 | 1.2 | 0.2 | 3.3 | 3.5 | 2.6 |
| Selectivity, mole % | 91 | 90 | 91 | 71 | 81 | 87 | 85 |

Example 5

A series of runs was carried out at 140°C to illustrate the ability of the present invention to produce the desired acetic acid at this low temperature. In contrast, the use of methyl iodide alone, rather than lithium iodide alone as in this invention, at 140°C results in little reaction under similar circumstances. The other reaction conditions and the recovery procedure were those described in Example 1. The reactants and results are summarized in Table IV.

TABLE IV

| Run | A | B | C | D | E |
|---|---|---|---|---|---|
| Reactor Charge | | | | | |
| $Rh(CO)_2$ AcAc, mmoles | ← | | 8 | | → |
| LiI, mmoles | 32 | 64 | 128 | 128 | 200 |
| Methyl formate, moles | ← | | 2.45 | | → |
| Reaction time, h | 3 | 3 | 3 | 5 | 3 |
| Initial CO pressure, (psig) bar | (540) 37.26 | (400) 27.6 | (400) 27.6 | (400) 27.6 | (400) 27.6 |
| Products Recovered (Liquid) | | | | | |
| Acetic acid, moles | 0.01 | 0.61 | 1.59 | 2.34 | 2.38 |
| Formic acid, moles | 0 | 0.03 | 0.06 | 0.04 | 0.02 |
| Methyl acetate, moles | 0 | 0.03 | 0.06 | 0.04 | 0.02 |
| Methyl formate, moles | 2.04 | 1.65 | 0.64 | 0.09 | 0.04 |
| Analytical Results | | | | | |
| Methyl formate conversion % | 27 | 32 | 75 | 96 | 95 |
| Conversion Rate, gmoles/l·h | 0.3 | 1.3 | 3.5 | 3 | 7 |
| Selectivity, % | 82 | 91 | 93 | 97 | 98 |

Example 6

In support of applicant's belief that the lithium iodide reacted at an extremely fast rate to cleave the formate ester and form lithium formate and organic iodide 2 runs were conducted using lithium iodide in the first and an amount of a mixture of lithium formate and methyl iodide equivalent thereto in the second to illustrate that the lithium iodide could be introduced by in situ production. This series was carried out under the conditions described in Example 1 and is summarized in Table V:

TABLE V

| Run | A | B |
|---|---|---|
| Reactor Charge | | |
| $Rh(CO)_2$ AcAc, mmoles | 8 | 8 |
| Methyl formate, moles | 2.45 | 2.45 |
| Lithium iodide, mmoles | 64 | — |
| Lithium formate, mmoles | — | 64 |
| Methyl iodide, mmoles | — | 64 |
| Products Recovered (Liquid) | | |
| Acetic acid, moles | 2.32 | 2.34 |
| Formic acid, moles | 0.01 | 0.03 |
| Methyl acetate, moles | 0.01 | 0.03 |
| Methyl formate, moles | 0 | 0.01 |
| Analytical Results | | |
| Methyl formate conversion, % | 99.7 | 99 |
| Conversion rate, gmoles/l·h | 5.1 | 5.1 |
| Selectivity, % | 98.4 | 99.1 |

Example 7

In this example the effect of ligand is shown. The reactions were carried out for 1 hour under the conditions described in Example 1. High conversion rate, selectivity and methyl formate conversion were observed. The results are summarized in Table VI:

TABLE VI

| Run | A | B | C | D |
|---|---|---|---|---|
| Reactor Charge | | | | |
| $Rh(CO)_2$ AcAc, mmoles | ← | 8 | | → |
| Lithium iodide, moles | ← | 64 | | → |
| Methyl formate, moles | ← | 2.45 | | → |
| Triphenylphosphine, mmoles | 16 | 32 | — | — |
| Triphenylamine, mmoles | — | — | 16 | — |
| Products Recovered (Liquid) | | | | |
| Acetic acid, moles | 1.66 | 1.34 | 1.94 | 1.64 |
| Formic acid, moles | 0.14 | 0.23 | 0.08 | 0.11 |
| Methyl acetate, moles | 0.14 | 0.23 | 0.08 | 0.11 |
| Methyl formate, moles | 0.52 | 0.66 | 0.4 | 0.49 |
| Analytical Results | | | | |
| Methyl formate conversion, % | 79 | 73 | 83 | 80 |
| Conversion rate, gmoles/l·h | 11 | 9 | 13 | 11 |
| Selectivity, % | 86 | 75 | 93 | 88 |

Example 8

For comparison purposes a series of experiments was carried out to compare and demonstrate the superiority of lithium iodide compared to methyl iodide. Under similar reaction conditions lithium iodide is far superior to methyl iodide when equivalent amounts are compared. The results are summarized in Table VII and were obtained using reaction condition similar to those described in Example 1.

TABLE VII

| Run | A | B | C | D |
|---|---|---|---|---|
| Reactor Charge | | | | |
| $Rh(CO)_2$ AcAc, mmoles | ← | | | → |
| Lithium iodide, moles | — | 32 | — | 128 |
| Methyl iodide, mmoles | 32 | — | 128 | — |
| Initial CO pressure, (psig) | (200) | (200) | (400) | (400) |
| bar | 13.8 | 13.8 | 27.6 | 27.6 |
| Products Recovered (Liquid) | | | | |
| Acetic acid, moles | 0 | 1.49 | 1.15 | 2.44 |
| Formic acid, moles | 0 | 0.15 | 0.16 | 0.04 |
| Methyl acetate, moles | 0 | 0.15 | 0.16 | 0.04 |
| Methyl formate, moles | 2.45 | 0.46 | 0.98 | 0 |
| Analytical Results | | | | |
| Methyl formate conversion, % | 0 | 81 | 60 | 99.9 |
| Conversion rate gmoles/l·h | 0 | 3.3 | 2.5 | 16.6 |
| Selectivity, % | 0 | 83 | 78 | 99.1 |

It should be noted that Runs A and C were carried out under conditions similar to those employed in examples shown in U.S.—A—4,194,056.

## Claims

1. A process for the production of organic carboxylic acids of the formula RCOOH by the catalytic reaction of an organic ester of formic acid of the formula HCOOR wherein R is an alkyl group having from 1 to 30 carbon atoms, or an aryl, aralkyl or alkaryl group having 6 or 10 ring carbon atoms with from 1 to 10 carbon atoms in the alk-moiety thereof, or an alkenyl group having from 2 to 30 carbon atoms, and CO or synthesis gas in contact with a catalytic system consisting essentially of rhodium or a rhodium compound and a halogen containing compound as promotor, characterized in that the catalytic system is a homogeneous system containing lithium iodide or bromide as promoter.

2. The process as claimed in claim 1 wherein the temperature is from 50°C to 350°C and the pressure is from 13.8 to 690 bar.

3. The process as claimed in claim 1 wherein the mole ratio of RH:LiI or LiBr is from 1 : 1 to 1 : 1,000 or from 1 : 8 to 1 : 150.

4. The process as claimed in claims 1 to 3 wherein the organic ester has (i) alkyl groups having from 1 to 15 carbon atoms, (ii) alkenyl groups having from 2 to 15 carbon atoms, or (iii) aryl, aralkyl or alkaryl groups having from 6 or 10 ring carbon atoms and from 1 to 4 carbon atoms in the alk-moiety thereof.

5. The process as claimed in claims 1 to 4 wherein the organic ester is methyl formate.

6. The process as claimed in claims 1 to 5 wherein an organic ligand of the formula $ER_3''$ is present, wherein E is nitrogen, phosphorus, arsenic, antimony and bismuth and R'' is an organic moiety.

7. The process as claimed in claim 6 wherein the ligand is a tertiary amine.

8. The process as claimed in claim 6 or 7 wherein the ligand is a phosphine.

**Patentansprüche**

1. Verfahren zur Herstellung organischer Carbonsäuren der Formel RCOOH durch katalytische Umsetzung eines organischen Esters der Ameisensäure der Formel HCOOR, worin R eine Alkylgruppe mit 1 bis 30 Kohlenstoffatomen oder eine Aryl-, Aralkyl- oder Alkarylgruppe mit 6 oder 10 Ring-Kohlenstoffatomen und 1 bis 10 Kohlenstoffatomen in der Alkylgruppe ist oder eine Alkenylgruppe mit 2 bis 30 Kohlenstoffatomen sein kann, mit CO oder Synthesegas in Gegenwart eines katalytischen Systems, bestehend im wesentlichen aus Rhodium oder einer Rhodiumverbindung und einer halogenhaltigen Verbindung als Promotor, dadurch gekennzeichnet, daß das katalytische System homogen ist und Lithiumiodid oder -bromid als Promotor enthält.

2. Verfahren nach Anspruch 1, worin die Reaktion bei einer Temperatur von 50 bis 350°C under einem Druck von 13,8 bis 690 bar erfolgt.

3. Verfahren nach Anspruch 1, worin das Molverhältnis RH:LiI oder LiBr 1 : 1 bis 1 : 1 000 oder 1 : 8 bis 1 : 150 beträgt.

4. Verfahren nach Anspruch 1 bis 3, worin der organische Ester (i) Alkylgruppen mit 1 bis 15 Kohlenstoffatomen, (ii) Alkenylgruppen mit 2 bis 15 Kohlenstoffatomen oder (iii) Aryl-, Aralkyl- oder Alkarylgruppen mit 6 oder 10 Ring-Kohlenstoffatomen und 1 bis 4 Kohlenstoffatomen in der Alkylgruppe aufweist.

5. Verfahren nach Anspruch 1 bis 4, worin der organische Ester Methylformiat ist.

6. Verfahren nach Anspruch 1 bis 5, worin ein organischer Ligandbildner der Formel $ER_3''$ vorhanden ist und E Stickstoff, Phosphor, Arsen, Antimon und Bismut und R'' eine organische Gruppe bedeuten.

7. Verfahren nach Anspruch 6, worin der Ligandbildner ein tertiäres Amin ist.

8. Verfahren nach Anspruch 6 oder 7, worin der Ligandbildner ein Phosphin ist.

**Revendications**

1. Procédé de production d'acides carboxyliques organiques de formule RCOOH par la réaction catalytique d'un ester organique d'acide formique de formule HCOOR, dans laquelle R est un groupe alkyle ayant 1 à 30 atomes de carbone ou un groupe aryle, aralkyle ou alkaryle ayant 6 ou 10 atomes de carbone cycliques avec 1 à 10 atomes de carbone dans leur portion alkyle, ou un groupe alcényle ayant 2 à 30 atomes de carbone, et de CO ou de gaz de synthèse au contact d'un système catalytique essentiellement formé de rhodium ou d'un composé de rhodium et d'un composé contenant un halogène comme promoteur, caractérisé en ce que le système catalytique est un système homogène contenant de l'iodure ou du bromure de lithium comme promoteur.

2. Procédé suivant la revendication 1, dans lequel la température va de 50 à 350°C et la pression va de 13,8 à 690 bars.

3. Procédé suivant la revendication 1, dans lequel le rapport molaire RH:LiI ou LiBr va de 1:1 à 1:1000 ou de 1:8 à 1:150.

4. Procédé suivant les revendications 1 à 3, dans lequel l'ester organique porte (i) des groupes alkyle ayant 1 à 15 atomes de carbone, (ii) des groupes alcényle ayant 2 à 15 atomes de carbone ou (iii) des groupes aryle, aralkyle ou alkaryle ayant 6 ou 10 atomes de carbone cycliques et 1 à 4 atomes de carbone, dans leur portion alkyle.

5. Procédé suivant les revendications 1 à 4, dans lequel l'ester organique est le formiate de méthyle.

6. Procédé suivant les revendications 1 à 5, dans lequel un ligand organique de formule $ER_3''$ est présent, E étant l'azote, le phosphore, l'arsenic, l'antimoine et le bismuth et R'' étant un groupement organique.

7. Procédé suivant la revendication 6, dans lequel le ligand est une amine tertiaire.

8. Procédé suivant la revendication 6 ou 7, dans lequel le ligand est une phosphine.